# EUROPEAN PATENT APPLICATION

(11) **EP 1 507 006 A1**
(43) Date of publication of application: **16.02.2005**
(21) Application number: 03751420.5
(22) Date of filing: 09.10.2003
(51) Int. Cl.: C12N 15/12, C07K 14/705, C12N 5/10, C12P 21/02, G01N 33/50, G01N 33/15, A61K 38/16, A61P 13/12

(54) **METHOD OF SCREENING REMEDY FOR RENAL FAILURE**

(30) Priority: 11.10.2002 JP 2002298958
(71) Applicant: YAMANOUCHI PHARMACEUTICAL CO. LTD., Tokyo 103-8411 (JP)
(72) Inventor: ENJO, Kentaro, Yamanouchi Pharmaceutical Co. Ltd., Tsukuba-shi, Ibaraki 305-8585 (JP); KUROMITSU, Sadao, Yamanouchi Pharmaceutical Co.Ltd, Tsukuba-shi, Ibaraki 305-8585 (JP)
(74) Representative: HOFFMANN - EITLE
(86) International application number: PCT/JP2003/012967
(87) International publication number: WO 2004/033687

(57) **Abstract**

A screening tool and a convenient screening method for obtaining a renal failure treating agent, and a pharmaceutical composition for treating renal failure and a production method thereof are disclosed.

The aforementioned screening tool is a G protein coupling type receptor FGK which is a polypeptide capable of activating CTGF promoter, a functionally equivalent modified substance thereof or a homologous polypeptide, or a cell expressing the aforementioned polypeptide. The screening method is a method which employs inhibition of the aforementioned polypeptide as the index.

## Description

### Technical Field

This invention relates to a cell expressing orphan GPCR and a method for screening an agent for treating renal failure which uses thereof.

### Background of the Invention

Chronic dialysis patients due to renal diseases are increasing every year. Since this is a serious problem in terms of medical economy too, great concern has been directed toward the improvement of its progress. The causes of renal disorders are various such as chronic glomerular nephritis, diabetic nephropathy and hypertensive nephrosclerosis, and origins thereof also are various such as an immunological mechanism and hypertension. However, irrespectively the cause, a common developing mechanism is considered in glomerular disorders at a certain stage. This is a point of view based on the excess filtration (hyperfiltration) theory proposed by Brenner *et al*. (Non-patent Reference 1). Outline of the hyperfiltration theory is described in the following, that is, a state of so-called glomerular hypertension is induced by the increase of glomerular pressure caused by a change in the hemodynamics in the kidney due to functional nephron reduction, hypertension, hyperglycemia, excessive protein ingestion and the like resulting from glomerulus disorders. This induces a glomerular cell disorder. As a result, it accelerates character conversion of mesangial cells and production of extracellular matrices and further develops into glomerulosclerosis. When functional nephron is reduced by this, glomerular pressure of the remaining functional nephron is further increased. The hypertrophy of remnant glomeruli occurs to compensate the function-lost nephrons in the beginning, but finally causes physical failure. The glomerulosclerosis is acceleratively progressed by this vicious circle and results in the last phase renal failure. This glomerulosclerosis and the production acceleration of extracellular matrices in the renal tubulointerstial cell are generally called renal fibrosis which is a histological change that coincides with the progress of renal failure which is irrespective of the original disease (Non-patent Reference 2). Accordingly, although it is needless to say that the treatment of individual original disease is important, it is considered that inhibition of the fibrosis as a final common pathway is an effective therapeutic approach.

It is known that various cytokines and growth factors are concerned in the fibrosis, and it is considered that TGF-β among them is the most important progressing factor since, for example, it induces production of extracellular matrix constituting proteins such as fibronectin and type I collagen(Non-patent Reference 3) and inhibits expression/function of enzymes degrading the extracellular matrix (Non-patent Reference 4). In addition, inhibition of the increase of extracellular matrices of the kidney by the expression/function inhibition of TGF-β using a TGF-β neutralizing antibody (Non-patent Reference 5), an antisense oligonucleotide (Non-patent Reference 6) decholin (Non-patent Reference 7) or a TGF-β receptor neutralizing antibody (Non-patent Reference 8) has been shown by nephritic animal models and its effectiveness has already been revealed. Thus, it has been shown that the inhibition of TGF-β signal leads to the renal failure treatment based on inhibition of renal fibrosis as the mechanism. However, since TGF-β is an important cytokine which has anti-inflammatory action and tumor growth inhibiting activity in addition to the fibrosis accelerating activity, and TGF-β knockout mice die of autoimmune disease (Non-patent Reference 9), it was considered that for the inhibition of fibrosis, inhibition of a factor which mediates fibrosis acceleration activity of TGF-β at its downstream is more desirable than direct inhibition of TGF-β (Non-patent Reference 10).

In recent year, a new cytokine connective tissue growth factor(CTGF) whose expression is induced by TGF-β was discovered (Non-patent Reference 11), and it has been reported that production of an extracellular matrix is induced by CTGF expression (Non-patent Reference 12), that expression of collagen induced by TGF-β is inhibited by a CTGF antisense oligonucleotide and an anti-CTGF antibody (Non-patent Reference 13), that expression of CTGF is increased in a human renal fibrosis pathologic tissue image (Non-patent Reference 14), that expression of CTGF is increased together with TGF-β in a rat pathological model kidney(Non-patent Reference 15), and that hepatocyte growth factor inhibits fibrosis of a mouse pathological model kidney via inhibition of CTGF production (Non-patent Reference 16). When these were generalized, a mechanism in which TGF-β induces expression of CTGF in the renal tissue, and CTGF further accelerates production of extracellular matrices together with TGF-β, thereby accelerating the fibrosis, has been revealed (Non-patent Reference 17). That is, it has been revealed that CTGF is a cytokine positioned at the downstream of TGF-β in the renal fibrosis, showing that it can become a new therapeutic target (Non-patent Reference 18). In addition, relation to pathological states has been pointed out such as that type I collagen whose expression is induced by TGF-β and CTGF shows low expression in human normal kidney and its expression increased in the glomerulus and tubular epithelium of pathological state kidney (Non-patent Reference 19), and that expression of type I collagen is increased by the induction of intrinsic TGF-β expression by high glucose in cultured mouse mesangial cells (Non-patent Reference 20). On the other hand, CTGF is expressed in broad range of tissues such as brain, placenta, lung, liver, kidney, skeletal muscle and the like, and its expression has no tissue specificity (Non-patent Reference 22).

FGK (fibrogenic GPCR in kidney) is an orphan GPCR identical to the GPR91 reported in 2001 (Non-patent Reference 21). Human GPR91 and mouse GPR91 are polypeptides respectively consisting of 330 and 317 amino acids, and it is considered that they are seven times transmembrane type receptors having 7 transmembrane regions. The human and mouse GPR91 molecules mutually have a homology of about 68%. Expression of the human GPR91 is found only in the kidney, and expression of the mouse GPR91 is slightly found in the liver in addition to the kidney. Although it has been reported that UTP functions as a ligand of human GPR91 in a reaction system which uses *Xenopus* egg (Patent Reference 1), its G protein coupling with GPR91 and physiological function remain unknown. Although sequences having homology with FGK are disclosed in Patent References 2 to 9, there is only description of information on expression other than that they are adenosine receptors (Patent Reference 9) and a P2U2 purinergic receptors (Patent Reference 8). Thus, their physiological functions are not known.

### (Patent Reference 1)

International Publication No. 97/20045 pamphlet

### (Patent Reference 2)

International Publication No. 97/24929 pamphlet

### (Patent Reference 3)

International Publication No. 01/98351 pamphlet

### (Patent Reference 4)

International Publication No. 00/22131 pamphlet

### (Patent Reference 5)

International Publication No. 01/90304 pamphlet

### (Patent Reference 6)

International Publication No. 00/31258 pamphlet

### (Patent Reference 7)

International Publication No. 02/00719 pamphlet

### (Patent Reference 8)

International Publication No. 02/61087 pamphlet

### (Patent Reference 9)

US No. 02/137887

### (Non-patent Reference 1)

*The New England Journal of Medicine,* (USA), 1982, vol.307, pp.652 - 659

### (Non-patent Reference 2)

*Journal of the American Society of Nephrology,* (USA), 1996, vol.7, pp.2495 - 2508

### (Non-patent Reference 3)

*The Journal of Biological Chemistry,* (USA), 1987, vol.262, pp.6443 - 6446

### (Non-patent Reference 4)

*Journal of the American Society of Nephrology,* (USA), 1999, vol.10, pp.790 - 795

### (Non-patent Reference 5)

Nature, (England), 1990, vol.346, pp.371 - 374

### (Non-patent Reference 6)

*Kidney International,* (USA), 1996, vol.50, pp.148 - 155

### (Non-patent Reference 7)

*Nature,* (England), 1992, vol.360, pp.361 - 364

### (Non-patent Reference 8)

*Kidney International,* (USA), 2001, vol.60, pp.1745 - 1755

### (Non-patent Reference 9)

*Nature,* (England), 1992, vol.359, pp.693 - 699

### (Non-patent Reference 10)

*Kidney International,* (USA), 1997, vol.51, pp.1388 - 1396

### (Non-patent Reference 11)

*Molecular Biology of the Cell,* (USA), 1993, vol.4, pp.637 - 645

### (Non-patent Reference 12)

*The Journal of Investigative Dermatology,* (USA), 1996, vol.107, pp.404 - 411

### (Non-patent Reference 13)

*The FASEB Journal,* (USA), 1999, vol.13, pp.1774 - 1786

### (Non-patent Reference 14)

*Kidney International,* (USA), 1998, vol.53, pp.853 - 861

### (Non-patent Reference 15)

*American Journal of Physiology Renal Physiology,* (USA), 2002, vol.282, pp.F933 - F942

### (Non-patent Reference 16)

*The FASEB Journal,* (USA), 2003, vol.17, pp.268 - 270

### (Non-patent Reference 17)

*Journal of the American Society of Nephrology,* (USA), 2001, vol.12, pp.472 - 484

### (Non-patent Reference 18)

*Kidney International,* (USA), 2000, vol.58, pp.1389 - 1399

### (Non-patent Reference 19)

*Kidney International,* (USA), 2002, vol.62, pp.137 - 146

### (Non-patent Reference 20)

*The Journal of Clinical Investigation,* (USA), 1994, vol.93, pp.536 - 542

### (Non-patent Reference 21)

*Journal of Molecular Biology,* (England), 2001, vol.307, pp.799 - 813

### (Non-patent Reference 22)

*Circulation,* (USA), 1997, vol.95, pp.831 - 839

### Disclosure of the Invention

As a result of intensive studies, the inventors of the present invention have found that FGK which is an orphan GPCR is expressed in kidney-specifically and activates the promoter of CTGF which is a therapeutic target of renal failure. Based on the knowledge, a method for screening a substance capable of inhibiting CTGF expression using FGK inhibition as a marker, namely a method for screening an agent for treating renal failure based on the CTGF expression inhibition by selecting a FGK inhibitor, was constructed. In addition, it was found that FGK, even by itself alone, can be used as a screening tool for an agent for treating renal failure using a change of its activity, and found that an inverse agonist of FGK surely inhibits expression of CTGF. Based on this, a method for screening an agent for treating renal failure by selecting an FGK inverse agonist was established. As described in the foregoing, since CTGF is expressed in a broad range of tissues and its expression has no tissue specificity, when a screening is carried out simply using the promoter region of CTGF gene, the resulting substance inhibits production of CTGF not only in the kidney but also in all tissues, so that there is a danger of causing side-effects based on the inhibition of cell growth and extracellular matrix production as the actions of CTGF. On the other hand, when a system for screening a substance capable of inhibiting CTGF expression using FGK inhibition as a marker which is constructed based on the knowledge found by the inventors of the present invention that FGK activates the CTGF promoter is used, since FGK is kidney-specifically expressed, it is expected that the inhibition of CTGF production by a resulting substance of the screening is kidney-specific and its expression in other tissues does not occur.

As a result of these, the inventors of the present invention have accomplished the invention by providing a convenient method for screening an agent for treating renal failure, and a pharmaceutical composition for treating renal failure and a production method thereof.

That is, the invention relates to,
(1) A screening tool for an agent for treating renal failure, which is a polypeptide consisting of the amino acid sequence represented by SEQ ID NO:2, or a polypeptide comprising an amino acid sequence represented by SEQ ID NO:2 in which from 1 to 10 amino acids are deleted, substituted and/or inserted and which is capable of activating CTGF promoter.
(2) The screening tool for an agent for treating renal failure, which is a cell expressing the polypeptide described in (1).
(3) A method for detecting whether or not a test compound is an inverse agonist, which comprises
   a step of allowing the cell described in (2) co-expressing a chimeric G protein in which C-terminal amino acid sequence is the amino acid sequence represented by SEQ ID NO:16, and which is a chimera of a partial polypeptide having promoting activity of phospholipase C activity of a phospholipase C activity-promoting G protein with a partial polypeptide having a Gi receptor coupling activity, to contact with a test compound, and
   a step of analyzing a change in activity of the polypeptide described in (1) in said cell.
(4) A method for screening an agent for treating renal failure, which comprises
   a step of allowing the cell described in (2) co-expressing a chimeric G-protein in which C-terminal amino acid sequence is the amino acid sequence represented by SEQ ID NO:16, and which is a chimera of a partial polypeptide having promoting activity of phospholipase C activity of a phospholipase C activity-promoting G protein with a partial polypeptide having a Gi receptor coupling activity, to contact with a test compound, and
   a step of analyzing a change in activity of the polypeptide described in (1) in said cell.
(5) A method for screening a substance inhibiting expression of CTGF, which comprises
   a step of allowing the cell described in (2) expressing the DNA of SEQ ID NO:13 having a reporter gene in downstream to contact with a test compound, and
   a step of measuring the reporter activity in said cell.
(6) The screening method according to (5), wherein the substance inhibiting expression of CTGF is an agent for treating renal failure.
(7) A method for screening an agent for treating renal failure, which comprises
   a step of allowing the cell described in (2) expressing the DNA of SEQ ID NO:14 having a reporter gene in downstream to contact with a test compound, and
   a step of measuring the reporter activity in said cell.
(8) A pharmaceutical composition for treating renal failure, which comprises an inverse agonist for the polypeptide described in (1).
(9) A pharmaceutical composition for treating renal failure, which comprises a substance obtainable by the method according to one of (4) to (7).
(10) A method for producing a pharmaceutical composition for treating renal failure, which comprises
   a step of screening using the method according to one of (4) to (7), and
   a step of preparing a pharmaceutical composition using a substance obtained by the screening.
(11) A method for treating renal failure, which comprises administering an effective amount of an inverse agonist for the polypeptide described (1) and/or a substance obtaiable by the method according to one of (4) to (7) to a subject in need of the treatment of renal failure.
(12) Use of an inverse agonist for the polypeptide described in (1) and/or a substance obtainable by the method according to one of (4) to (7) for the manufacture of a pharmaceutical composition for treating renal failure.

Patent Reference 1 describes a receptor having the same sequence of FGK which is a polypeptide as the screening tool of the present invention. It is described that this is expressed in the kidney and activated by ATP, ADP, UTP and UDP and that this is useful as a tool for screening. However, there is no description regarding its usefulness as a tool for what object of the screening, and its illustrative use. Patent Reference 2 to Patent Reference 9 disclose molecules having homology with FGK. Patent Reference 6 shows that the molecule is expressed in the kidney, but there is no description regarding its illustrative use. Although in Patent Reference 7, renal disease and renal failure are included in various diseases cited to which the molecule relates, there is no description supporting the same. Patent Reference 8 discloses a large number of GPCR including molecules having high homology with FGK, and a large number of diseases in which a large number of GPCR are concerned. Although renal disease is included therein, their illustrative examples are not described and there are no experimental supports on their use. Patent Reference 9 discloses a receptor having homology with FGK and discloses a method for identifying agonists or antagonists thereof. It is described that these agonists and antagonists are useful for treating vasodilatation, hypotension, chronic renal disease, thyroid disease and immune diseases including asthma. However, it only describes that the molecule is expressed in specific tissues including the kidney as the basis that they are useful for treating chronic renal disease, and does not describes the relationship between the molecule and CTGF. Relationship between a molecule having homology with FGK and renal disease and relationship between the molecule and CTGF are not described in any of Patent References 2 to 5.

Accordingly, the fact that FGK activates the promoter of CTGF which is a therapeutic target of renal failure is novel knowledge found by the inventors of the present invention, and the method for screening a substance capable of inhibiting CTGF expression using FGK inhibition as a marker, the method for screening an agent for treating renal failure by selecting a FGK inverse agonist, and the pharmaceutical composition for treating renal failure and the production method thereof are inventions provided for the first time by the inventors of the present invention.

### Brief Description of the Drawings

Fig. 1 is a graph showing luciferase activity when pCTGF-luc or pCOLIA2-luc reporter plasmid was transfected into HEK293 cells, and changes in the activity caused by FGK gene cotransfection. Ordinate of the graph shows relative value of the luciferase activity.
Fig. 2 is a graph showing luciferase activity when pEF-BOS-Gqi and pSRE-luc reporter plasmids were cotransfected into HEK293 cells, and changes in the activity caused by FGK gene transfection. Ordinate of the graph shows relative value of the luciferase activity.

### Best Mode for Carrying Out the Invention

The following describes the invention in detail.

Firstly, the terms used in the present invention are described.

The "FGK" as used herein means "FGK protein", and the "inverse agonist" represents a substance which inhibits spontaneous activity (namely an activity detected by activated FGK existing in a equilibrium state in the absence of a FGK ligand or agonist) of a polypeptide for screening of the invention (e.g., FGK). The method for judging whether or not a polypeptide "activates the CTGF promoter" is not particularly limited, but it can be judged for example by verifying whether or not the polypeptide shows a dose-dependent transactivation under the conditions described in Example 3. The "Gi" is one of the subfamily of G protein which functions as a signal transduction amplifying factor into cells by coupling with a receptor, and is a G protein that inhibits the activity of adenylate cyclase. When the adenylate cyclase activity is inhibited, for example, concentration of intracellular cAMP is reduced. The "phospholipase C activity-promoting G protein" is one of the subfamily of G protein which functions as a signal transduction amplifying factor into cells by coupling with a receptor, and is a G protein which promotes the activity of phospholipase C. When the activity of phospholipase C is promoted, for example, intracellular Ca²⁺ concentration increases. As the phospholipase C activity promoting G protein, for example, Gq can be cited.

### <Screening tool for an agent for treating renal failure>

A screening tool of the present invention for an agent for treating renal failure includes a polypeptide type screening tool for an agent for treating renal failure and a cell type screening tool for an agent treating renal failure. The "screening tool" as used herein means a substance to be used for the screening (illustratively a polypeptide or a cell expressing the polypeptide, which is used in the screening). The "screening tool for an agent treating renal failure" is a polypeptide or cell as the subject to be contacted with a test compound in the method for screening an agent for treating renal failure of the present invention, for screening an agent for treating renal failure. Use of the aforementioned polypeptide described in [1] or cell described in [2] for the screening of an agent for treating renal failure is also included in the present invention.

### (1) Polypeptide type screening tool for an agent for treating renal failure

The polypeptide type screening tool for an agent for treating renal failure includes
1) the screening tool for an agent for treating renal failure which is a polypeptide consisting of the amino acid sequence represented by SEQ ID NO:2;
2) the screening tool for an agent for treating renal failure which is a polypeptide that comprises an amino acid sequence in which from 1 to 10 amino acids of the amino acid sequence represented by SEQ ID NO:2 are deleted, substituted and/or inserted and which can activate CTGF promoter (to be referred to as functionally equivalent modified substance hereinafter); and
3) the screening tool for an agent for treating renal failure which is a polypeptide that comprises an amino acid sequence having 90% or more of homology with the amino acid sequence represented by SEQ ID NO:2 and which can activate CTGF promoter (to be referred to as homologous polypeptide hereinafter).

Among the polypeptide type screening tools for an agent for treating renal failure, the polypeptide consisting of the amino acid sequence represented by SEQ ID NO:2 is a member of the commonly known human orphan GPCR, and the polypeptide is called human FGK.

As the functionally equivalent modified substance which can be used as a polypeptide type screening tool for an agent for treating renal failure, "a polypeptide which comprises an amino acid sequence in which from 1 to 10, preferably from 1 to 7, more preferably from 1 to 5, amino acids of the amino acid sequence represented by SEQ ID NO:2 are deleted, substituted and/or inserted and which can activate CTGF promoter" is preferable.

Among the homologous polypeptides, a protein which comprises an amino acid sequence having a homology of preferably 90% or more, more preferably 95% or more, further preferably 98% or more, regarding the amino acid sequence represented by SEQ ID NO:2 is preferable, and more preferably which can activate CTGF promoter. In this connection, the aforementioned "homology" as used herein means a value (Identities) obtained by Clustal program (Higgins and Sharp, Gene, vol. 73, pp. 237 - 244, 1998; Thompson *et al*., *Nucleic Acid Res*., vol. 22, pp. 4673 - 4680, 1994) retrieval using parameters prepared by default. The aforementioned parameters are as follows.

As Pairwise Aliment Parameters
K tuple 1
Gap Penalty 3
Window 5
Diagonals Saved 5

Various polypeptides which can be used as the polypeptide type screening tool for an agent for treating renal failure, namely human FGK, functionally equivalent modified substances and homologous proteins, are hereinafter called polypeptides for a screening tool.

Not only mutants in human but also FGK or mutants thereof derived from organisms other than human (e.g., mouse, rat, hamster or dog) are included in the polypeptides for a screening tool. Proteins artificially modified by genetic engineering techniques based on these natural proteins (that is, human derived mutants, or FGK derived from organisms other than human or mutants thereof) or human FGK are also included. In this connection, the "mutant" (variation) as used herein means an individual difference found in the same protein within the same species, or a difference found in homologous protein among several species.

The mutants of human FGK in human, or FGK derived from organisms other than human or mutants thereof, can be obtained by those skilled in the art based on the information on a nucleotide sequence of human FGK gene (e.g., the nucleotide sequence represented by SEQ ID NO:1). In this connection, unless otherwise noted, the genetic engineering techniques can be carried out in accordance with the conventionally known methods (Maniatis, T. *et al*., "Molecular Cloning - A Laboratory Manual", Cold Spring Harbor Laboratory, NY, 1982, and the like).

For example, a desired protein can be obtained by designing appropriate primers or probe based on the information on the nucleotide sequence of human FGK gene, carrying out PCR or hybridization using the aforementioned primers or probe and a sample (e.g., a total RNA or mRNA fraction, a cDNA library or a phage library) derived from a organism of interest [e.g., a mammal (e.g., human, mouse, rat, hamster or dog)], thereby obtaining a gene of the protein, expressing the gene using an appropriate expression system, and then confirming that the thus expressed protein can activate the CTGF promoter by, for example, the method described in Example 3.

In addition, regarding the aforementioned proteins artificially modified by genetic engineering techniques, a desired protein can be obtained by obtaining a gene of the protein by a usual method, for example, site-specific mutagenesis (Mark, D.F. *et al., Proc. Natl. Acad. Sci. USA*, **81,** 5662 - 5666, 1984), expressing the gene using an appropriate expression system, and then confirming that the thus expressed protein can activate the CTGF promoter by, for example, the method described in Example 3.

The polypeptide for a screening tool can be obtained by various conventionally known methods, for example, it can be prepared by conventionally known genetic engineering techniques using a gene coding for the protein of interest. More illustratively, it can be prepared by culturing a cell or transformed cell (that is, a transformed cell which is transfected with an expression vector containing a DNA coding for the polypeptide for a screening tool and expressing the aforementioned polypeptide) which is described later, under such a condition that the polypeptide for a screening tool can be expressed, and then separating and purifying the protein of interest from the culture mixture by methods generally used for the separation and purification of receptor proteins.

In preparing a polypeptide for a screening tool, the method for obtaining a gene encoding the same is not particularly limited, but for example, in case that human FGK is prepared, a DNA consisting of the nucleotide sequence represented by SEQ ID NO:1 can for example be used as the gene coding for the same. In this connection, the codons corresponding to a desired amino acid are well known by themselves, and their selection may be optional and, for example, they can be determined in accordance with a usual method by taking codon usage of the host to be used into consideration (Crantham, R. *et al*., *Nucleic Acids Res.,* **9,** r43 - r74, 1981).

The DNA consisting of the nucleotide sequence represented by SEQ ID NO:1 can be obtained, for example, by ligating DNA fragments produced by a chemical synthesis method, or can be obtained by the polymerase chain reaction (PCR) method (Saiki, R.K. *et al., Science,* **239,** 487 - 491, 1988) using a cDNA library derived from a cell or tissue having the ability to produce human FGK as the template and using an appropriate primer set designed based on the nucleotide sequence represented by SEQ ID NO:1. As the aforementioned cell or tissue having the ability to produce human FGK, for example, human kidney and the like can be cited. Also, as the aforementioned primer set includes such as a combination of an oligonucleotide consisting of the nucleotide sequence represented by SEQ ID NO:3 and an oligonucleotide consisting of the nucleotide sequence represented by SEQ ID NO:4.

Although the separation and purification methods which can be used in the preparation of a polypeptide for a screening tool are not particularly limited, they can be carried out, for example, by the following procedures. For example, a cell membrane fraction containing a polypeptide for a screening tool can be obtained by culturing a cell expressing the polypeptide for a screening tool on its surface, suspending them in a buffer, and then homogenizing to centrifuge them. The polypeptide for a screening tool can be purified by solubilizing the thus obtained cell membrane fraction, and then carrying out its treatment with a general protein precipitating agent, ultrafiltration, various types of liquid chromatography [e.g., molecular sieve chromatography (gel filtration), adsorption chromatography, ion exchange chromatography, affinity chromatography, high performance liquid chromatography (HPLC) and the like], dialysis, or a combination thereof. In this connection, characteristics of the receptor can be maintained even after the solubilization by the use of a solubilizing agent as mild as possible (e.g., CHAPS, Triton X-100, digitonin or the like) in solubilizing the cell membrane fraction.

When the polypeptide for the screening tool is prepared, confirmation of expression of the aforementioned polypeptide, confirmation of its intracellular localization, its purification or the like can be easily carried out by inframe-fusing a polypeptide for a screening tool with an appropriate marker sequence and expressing it, if necesssary. The aforementioned marker sequence includes FLAG epitope, hexa-histidine tag, hemagglutinin tag and myc epitope. In addition, by insertion of a specific sequence recognized by a protease (e.g., enterokinase, factor Xa, thrombin or the like) between the marker sequence and polypeptide for a screening tool, digestion and removal of the marker sequence part by such a protease is possible. For example, there is a report stating that muscarine acetylcholine receptor and hexa-histidine tag were connected with a thrombin recognizing sequence (Hayashi, M.K. and Haga, T. *J. Biochem.,* **120,** 1232 - 1238, 1996).

### (2) Cell type screening tool for an agent for treating renal failure

The cell type screening tool of the present invention for an agent for treating renal failure includes
1) a screening tool for an agent for treating renal failure which is a cell expressing human FGK;
2) a screening tool for an agent for treating renal failure which is a cell expressing a functionally equivalent modified substance; and
3) a screening tool for an agent for treating renal failure which is a cell expressing a homologous protein.

The cell which can be used as the cell type screening tool of the present invention for an agent for treating renal failure (to be referred to as cell for screening tool hereinafter) is not particularly limited, as far as it expresses the aforementioned polypeptide for a screening tool when used as a cell type screening tool for an agent for treating renal failure, and it can be a transformed cell in which the aforementioned polypeptide is artificially expressed, or it can be a natural cell or a cell strain thereof which is known to express a polypeptide for a screening tool. However, a transformed cell in which the aforementioned polypeptide is artificially expressed is preferable.

The host cell which can be used for preparing various types of transformed cell which can be used as the cell type screening tool of the present invention for an agent for treating renal failure(to be referred to as transformed cell for screening tool hereinafter) is not particularly limited, as far as it expresses a polypeptide for a screening tool, and its examples include generally used conventionally known microorganisms such as *Escherichia coli* or a yeast (*Saccharomyces cerevisiae*), or conventionally known cultured cells such as a vertebrate cell (e.g., CHO cell, HEK293 cell or COS cell) or an insect cell (e.g., Sf9 cell).

The aforementioned vertebrate cell includes such as COS cell as a monkey cell (Gluzman, Y., *Cell*, **23**, 175 - 182, 1981), a dihydrofolate reductase deficient strain of Chinese hamster ovary cell (CHO) (Urlaub, G and Chasin, L.A., *Proc. Natl. Acad. Sci. USA,* **77**, 4216 - 4220, 1980), a human fetal kidney-derived HEK293 cell, or a 293-EBNA cell (Invitrogen) in which Epstein-Barr virus EBNA-1 gene is transferred into the aforementioned HEK293 cell.

The expression vector which can be used for preparing a transformed cell for a screening tool is not particularly limited, with as far as it can express a polypeptide for a screening tool, and it can be optionally selected in accordance with the kind of a host cell to be used.

For example, as the expression vector for vertebrate cells, those which have a promoter, a RNA spice site, a polyadenylation site, a transcription termination sequence and the like locating at the upstream of a gene to be expressed can generally be used, and they may further have a replication origin, if necessary. Example of the aforementioned expression vector includes such as pSV2dhfr having SV40 early promoter (Subramani, S. *et al., Mol*. *Cell. Biol*., **1,** 854 - 864, 1981), pEF-BOS having a human elongation factor promoter (Mizushima, S. and Nagata, S., *Nucleic Acids Res.,* **18,** 5322, 1990) and pCEP4 having cytomegalovirus promoter (Invitrogen).

More illustratively, when COS cell is used as the host cell, an expression vector which has the SV40 replication origin, can perform autonomous replication in COS cell, and further has a transcription promoter, a transcription termination signal and an RNA splice site can be used, and examples thereof include pME18S (Maruyama, K. and Takebe, Y., *Med. Immunol*., **20,** 27 - 32, 1990), pEF-BOS (Mizushima, S. and Nagata, S., *Nucleic Acids Res.,* **18**, 5322, 1990) and pCDM8 (Seed, B., *Nature,* **329,** 840 - 842, 1987).

The aforementioned expression vector can be incorporated into COS cell, by such as DEAE-dextran method (Luthman, H. and Magnusson, G., *Nucleic Acids Res.*, **11**, 1295 - 1308, 1983), calcium phosphate-DNA co-precipitation method (Graham, F.L. and van der Ed, A.J., *Virology,* **52,** 456 - 457, 1973), a method which uses a cationic liposome reagent (Lipofectamine; Gibco BRL), electroporation (Neumann, E. *et al., EMBO J.,* **1**, 841 - 845, 1982) or the like.

Also, when CHO cell is used as the host cell, a transformed cell which stably produces a polypeptide for a screening tool can be obtained by cotransfection of an expression vector containing a DNA coding for the polypeptide for a screening tool together with a vector capable of expressing a neo gene which functions as a G418 resistance marker, such as pRSVneo (Sambrook, J. *et al*, "Molecular Cloning - A Laboratory Manual", Cold Spring Harbor Laboratory, NY, 1989), or pSV2-neo (Southern, P.J. and Berg, P., *J. Mol. Appl. Genet.,* **1**, 327 - 341, 1982), and then selecting a G418-resistant colony.

In addition, when 293-EBNA cell is used as the host cell, pCEP4 (Invitrogen) or the like which has the replication origin of Epstein-Barr virus and can perform autonomous replication in 293-EBNA cell can be used as the expression vector.

The transformed cell for a screening tool can be cultured in accordance with the usual method, and a polypeptide for a screening tool is produced in the cell or on the cell surface. As the medium which can be used in the aforementioned culture, generally used various media can be optionally selected in accordance with the employed host cell. For example, in the case of COS cell, a medium such as Dulbecco's Modified Eagle's minimum essential medium (DMEM) or the like further supplemented with fetal bovine serum (FBS) or the like serum component as occasion demands can be used. Also, in the case of 293-EBNA cell, Dulbecco's Modified Eagle's minimum essential medium (DMEM) or the like medium supplemented with fetal bovine serum (FBS) or the like serum component can be used by further adding G418 thereto.

The transformed cell for a screening tool is not particularly limited so far as it expresses a polypeptide for a screening tool. It is desirable that the transformed cell for a screening tool expresses a G protein in which its C-terminal amino acid sequence is the amino acid sequence represented by SEQ ID NO;16 (Asp-Cys-Gly-Leu-Phe), in addition to the polypeptide for a screening tool. The amino acid sequence represented by SEQ ID NO;16 is an amino acid sequence consisting of the C-terminal 5 amino acid residues of Gi, and the "G protein in which its C-terminal amino acid sequence is the amino acid sequence represented by SEQ ID NO;16" is called "C terminal Gi type G protein" in the following.

The aforementioned C terminal Gi type G protein include such as (1) Gi or (2) a chimeric G protein in which its C-terminal amino acid sequence is the amino acid sequence represented by SEQ ID NO:16, and which is a chimera of a partial polypeptide having promoting activity of phospholipase C activity of a phospholipase C activity-promoting G protein (e.g., Gq) with a partial polypeptide having a Gi receptor coupling activity. In the following, the chimeric G protein of a partial polypeptide having a phospholipase C activity promoting activity of Gq with a partial polypeptide having a Gi receptor coupling activity is called Gqi.

The polypeptide for a screening tool binds to Gi by recognizing an amino acid sequence consisting of the C-terminal 5 amino acid residues of Gi (namely, the amino acid sequence represented by SEQ ID NO;16). Accordingly, the polypeptide for a screening tool can binds to not only Gi but also Gqi. When the polypeptide for a screening tool and C terminal Gi type G protein are expressed in the transformed cell for a screening tool, these polypeptides can be bonded together inside the cell.

The "partial polypeptide having promoting activity of phospholipase C activity of Gq" according to the aforementioned Gqi is not particularly limited, as far as that it does not contain the C terminal amino acid sequence and it has the promoting activity of phospholipase C activity. An example thereof includes an N terminal side partial polypeptide of Gq in which the amino acid sequence consisting 5 amino acid residues in C-terminal is deleted.

The "partial polypeptide having a Gi receptor conjugating activity" according to the aforementioned Gqi is not particularly limited, with as far as it contains the amino acid sequence consisting of 5 amino acid residues in the C-terminal of Gi and it does not have the activity to inhibit activity of adenylate cyclase. An example thereof includes a C terminal side partial polypeptide of Gi consisting of the amino acid sequence represented by SEQ ID NO:16.

### <Inverse agonist detection method>

Using the aforementioned polypeptide for a screening tool or cell for a screening tool as a detection tool, whether or not a test compound is an inverse agonist of the polypeptide for screening of the present invention (preferably FGK) can be detected. A polypeptide for screening which activates the promoter of CTGF as a target of an agent for treating renal failure (e.g., FGK) can be used as a screening tool for an agent for treating renal failure even by itself alone. An inverse agonist of the polypeptide for screening (preferably FGK) is an useful substance as an agent for treating renal failure. According to the detection method of the present invention, detection of a change of the activity of a polypeptide for screening (e.g., FGK) is carried out by measuring an index of activity according to a physiological characteristic of the protein to be used for the screening. The index is for example a change in the Ca²⁺ concentration or a change in the amount of cAMP. Illustratively, the detection methods described in the following can be exemplified. As the polypeptide for screening, a cell expressing the receptor, a membrane fraction of the cell, a purified preparation of the protein or the like can also be used.

The method of the present invention for detecting whether or not a test compound is an inverse agonist of the polypeptide for screening (preferably FGK) include
1) a method for detecting whether or not it is an inverse agonist of the polypeptide for screening (preferably FGK) using a change of intracellular Ca²⁺ concentration as an index (that is, a Ca²⁺ type detection method);
2) a method for detecting whether or not it is an inverse agonist of the polypeptide for screening (preferably FGK) using a change of the amount of intracellular cAMP as an index (that is, a cAMP type detection method); and
3) a method for detecting whether or not it is an inverse agonist of the polypeptide for screening (preferably FGK) using of a GTPγS binding method (to be referred to as GTPγS binding type detection method hereinafter). These detection methods are explaind in order.

### 1) Ca²⁺ type detection method

The Ca²⁺ type detection method of the invention uses a cell co-expressing (i) a polypeptide for a screening tool and (ii) a chimeric G protein (e.g., Gqi) in which its C-terminal amino acid sequence is the amino acid sequence represented by SEQ ID NO:16 and which is a chimera of a partial polypeptide having promoting activity of phospholipase C activity of a phospholipase C activity-promoting G protein with a partial polypeptide having a Gi receptor coupling activity (to be referred to as cell for Ca²⁺ type detection hereinafter). When whether or not it is an inverse agonist is detected by the Ca²⁺ type detection method of the present invention, the cell for Ca²⁺ type detection is allowed to contact with a test compound, and a change in the Ca²⁺ concentration inside the aforementioned cell for Ca²⁺ type detection is directly or indirectly analyzed (namely, measured or detected). Regarding the change in Ca²⁺ concentration, a change in the Ca²⁺ concentration can be directly analyzed using, for example, a calcium binding fluorescence reagent (e.g., fura2 or fluo3 or the like), or a change in the Ca²⁺ concentration can be indirectly analyzed by analyzing the transcriptional activity of a gene in which its transcriptional level is controlled depending on the Ca²⁺ concentration [e.g., a gene in which an activator protein 1 (AP1) responding sequence is inserted into upstream of a luciferase gene].

When that the cell for Ca²⁺ type detection is allowed to contact with a test compound, if Ca²⁺ concentration in the cell for Ca²⁺ type detection is reduced, it can be judged that it is an inverse agonist of the polypeptide for screening (preferably FGK). In this connection, it is desirable to carry out the same operation using a control cell in which the polypeptide for a screening tool is not expressed but Gqi is expressed, or the host cell before transformation as a control instead of the cell for Ca²⁺ type detection co-expressing the polypeptide for a screening tool, and Gqi and thereby to confirm that the intracellular Ca²⁺ concentration of the aforementioned control cell or the aforementioned host cell is not reduced.

More illustratively, this can be carried out making use of the method of Example 4 or Example 5.

As described above, Gi is not directly used but Gqi is used as the coupling protein in the Ca²⁺ type detection method of the present invention, so that the detection of whether or not a sample is an inverse agonist can be carried out by analyzing not the cAMP concentration but the Ca²⁺ concentration. In general, the Ca²⁺ concentration can be measured more conveniently and quickly in comparison with the cAMP concentration.

### 2) cAMP type detection method

In the cAMP type detection method of the present invention, a cell for a screening tool is used as the cell for cAMP type detection. Since Gi is constitutively expressed in general host cells, a cell for cAMP type detection can be obtained by using a natural cell or a cell strain thereof which is known to express a polypeptide for a screening tool, or by transforming a host cell with an expression vector containing a DNA coding for a polypeptide for a screening tool.

When whether or not it is an inverse agonist is detected by the cAMP type detection method of the present invention, the cell for cAMP type detection is allowed to contact with a test compound, and a change in the cAMP concentration inside the aforementioned cell for cAMP type detection is directly or indirectly analyzed (namely, measured or detected). With regard to the change in cAMP concentration, a change in the cAMP concentration can be directly analyzed using, for example, a commercially available cAMP measuring kit (Amersham or the like), or a change in the cAMP concentration can be indirectly analyzed by analyzing the transcription activity of a gene in which its transcriptional level is controlled depending on the cAMP concentration [e.g., a gene in which a cAMP responsive element (CRE) is inserted into upstream of a luciferase gene].

In case that the cell for cAMP type detection is allowed to contact with a test compound, when cAMP concentration inside the cell for cAMP type detection is increased, it can be judged that the aforementioned test compound is an inverse agonist for FGK. Also, it is desirable to carry out the same operation using a cell in which the polypeptide for a screening tool is not expressed as a control, instead of the cell for cAMP type detection expressing the polypeptide for a screening tool and Gi, and thereby to confirm that the cAMP concentration inside the aforementioned cell is not increased by the aforementioned test compound. More illustratively, for example, this can be carried out by detecting whether or not the cAMP concentration is increased when a test compound is contacted, in comparison with a case when the test compound is not contacted when a low dose (e.g., 0.2 µg) of FGK is expressed under the same condition of Example 4.

### 3) GTPγS binding type detection method

By the GTPγS binding type detection method of the present invention, whether or not a sample is an inverse agonist for FGK can be detected, using the GTPγS binding method (Lazareno, S. and Birdsall, N.J.M., *Br. J. Pharmacol.,* **109,** 1120 - 1127, 1993) in which a polypeptide for a screening tool, a cell membrane fraction containing the aforementioned polypeptide or a cell expressing the aforementioned polypeptide are used. A cell or cell membrane fraction for GTPγS binding type detection method (a cell for GTPγS type detection) can be obtained by using a natural cell or a cell strain thereof which is known to express a polypeptide for a screening tool, or by transforming a host cell with an expression vector containing a DNA coding for a polypeptide for a screening tool.

For example, this can be carried out by the following procedure.

That is, a cell membrane containing a polypeptide for a screening tool is mixed with ³⁵S-labeled GTPγS (400 pmol/L) in a mixed solution of 20 mmol/L HEPES (pH 7.4), 100 mmol/L NaCl, 10 mmol/L MgCl₂ and 50 mmol/L GDP. After incubation in the presence or absence of a test compound, the reaction mixture is filtered through a glass filter or the like, and the radioactivity of GTPγS remained on the filter is measured using a liquid scintillation counter or the like. Whether or not the sample is an inverse agonist for FGK can be detected by using reduction of the specific GTPγS binding in the presence of the test compound as an index. Also, it is desirable to carry out the same operation using a cell membrane in which the polypeptide for a screening tool is not expressed as a control, instead of the cell membrane expressing the polypeptide for a screening tool, and thereby to confirm that the GTPγS binding is not reduced in the presence of the aforementioned test compound.

### <Method for screening a substance which inhibits CTGF expression>

A method for screening a substance which inhibits CTGF expression, using inhibition of activity of a polypeptide for screening (preferably FGK) as an index is included in the present invention. A reporter assay system which uses the promoter region of a CTGF gene can be used in the method. As already described above, it has been revealed that CTGF is a cytokine locating downstream of TGF-β in renal fibrosis, showing that CTGF is shown to be a target for therapeutic for an agent for treating renal failure. The inventors of the present invention have found that FGK activates the CTGF promoter considered to be the target for a drug creation for renal failure as shown in Example 3 which is described later, and further established a method for screening a substance which inhibits CTGF expression, using activity inhibition of FGK activity as an index.

The method of the present invention for screening a substance which inhibits CTGF expression with the feature comprising
a step of allowing a cell for a screening tool expressing the DNA (CTGF promoter) of SEQ ID NO:13 having a reporter gene in its downstream to contact with a test compound, and
a step of measuring the reporter activity in the aforementioned cell, and a substance which inhibits expression of CTGF can be screened.

As the cell to be used in the method, a cell coexpressing (i) a polypeptide for a screening tool and (ii) a reporter gene fused with a CTGF promoter region (to be referred to as a cell for CTGF promoter type detection hereinafter) can be used. In this connection, with regard to the aforementioned cell, it is desirable that the host cell before subjecting to the transformation is a cell derived from the kidney. As such cell, for example, the aforementioned HEK293 cell can be cited.

The reporter gene assay (Tamura *et al*., Method for Studying Transcription Factors (written in Japanese), published by Yodo-sha, 1993) is a method for detecting expression regulation of genes using expression of a reporter gene as the marker. Expression regulation of a gene is generally controlled at a site called promoter region existing in its upstream of 5' end, and the quantity of gene expression at the transcription stage can be estimated by measuring the activity of this promoter. When a test substance activates the promoter, it activates transcription of a reporter gene located in the downstream of the promoter region. In this manner, the promoter activation activity, namely the expression acceleration activity, can be detected by replacing it with the expression of the reporter gene. Accordingly, the activity of a test compound on the expression regulation of CTGF can be detected by the reporter gene assay using the CTGF promoter region, by replacing it with the expression of the reporter gene. The "reporter gene" fused with the CTGF promoter region consisting of the nucleotide sequence represented by SEQ ID NO:16 is not particularly limited so long as it is a generally used one, but an enzyme gene or the like which can be quantitatively easily measured is desirable. For example, a bacterial transposon-derived chloramphenicol acetyltransferase gene (CAT), a firefly-derived luciferase gene (Luc) a jellyfish-derived green fluorescent protein gene (GFP) and the like can be cited. The reporter gene should be functionally fused with the promoter region of CTGF consisting of the nucleotide sequence represented by SEQ ID NO:16. Test compound-dependent changes in the induction of transactivation can be analyzed by comparing expression quantities of the reporter gene when the cell for CTGF promoter type detection is allowed to contact and not allowed to contact with a test compound.

It is desirable to carry out the same operation using a cell for a screening tool in which the polypeptide for a screening tool is not expressed but the reporter gene fused with the CTGF promoter region is expressed as a control, instead of the cell for CTGF promoter type detection and thereby to confirm that the reporter activity in the aforementioned cell for a screening tool is not inhibited by the aforementioned test compound.

By carrying out the aforementioned process and selecting a substance which inhibits the reporter activity, screening of a substance capable of inhibiting expression of CTGF can be carried out. Illustratively, the aforementioned screening can be carried out by the method described in Example 3. For example, by adding a sets compound under the assay condition described in Example 3, a substance having an IC₅₀ value of 10 µM or less, preferably a substance having an IC₅₀ value of 1 µM or less, more preferably a substance having an IC₅₀ value of 0.1 µM or less, under the condition of Example 3 can be selected as a substance having the activity to inhibit CTGF expression. More preferably, this can be carried out in the same manner under the condition of Example 7.

### <Method for screening an agent for treating renal failure>

An agent for treating renal failure can be screened using the screening tool for an agent for treating when the renal failure of the present invention (includes both of the polypeptide type screening tool for an agent for treating renal failure and cell type screening tool for an agent for treating renal failure).

As already described, it has been revealed that CTGF is a cytokine locating downstream of TGF-β in renal fibrosis, showing that CTGF is shown to be a target for therapeutic for an agent for treating renal failure, namely a substance which inhibits expression of CTGF is shown to be an agent for treating renal failure. Also, it has been revealed that FGK is localized in the kidney. Additionally, the inventors of the present invention have found that FGK activates the CTGF promoter and also activates the human type I collagen alpha two subunit (COLIA2) promoter as described in Example 3. According to these findings, an inverse agonist of a polypeptide for screening (preferably FGK) or a substance which inhibits expression of CTGF by inhibiting FGK activity is useful as an agent for treating renal failure. Thus, the polypeptide for a screening tool itself or cell for a screening tool itself so far described can be used as a screening tool for the screening of an agent for treating renal failure.

The test compounds which can be subjected to screening using a screening tool for an agent for treating renal failure of the present invention are not particularly limited. For example, various conventionally known compounds (including peptides) registered in the chemical file, a group of compounds obtained by combinatorial chemistry techniques (Terrett, N.K. et al., *Tetrahedron,* **51,** 8135-8137, 1995) or a group of random peptides prepared by applying the phage display method (Felici, F. *et al*., *J. Mol. Biol*., **222,** 301 - 310, 1991) and the like can be used. Also, microbial culture supernatants, natural components of plants or marine organisms, or animal tissue extracts and the like can be used as test compounds of the screening. Additionally, compounds (including peptides) obtained by chemically or biologically modifying the compounds (including peptides) selected by the screening tool for an agent for treating renal failure of the present invention can be used.

The screening method of the present invention is roughly divided into the following three types based on the respective detection methods, and a substance useful as an agent for treating renal failure can be screened using any one of these method or a combination thereof. The screening methods of the present invention:
(1) a screening method which uses activity change of a polypeptide for screening (preferably FGK) as the index,
(2) a method for screening a substance which inhibits CTGF expression, using activity inhibition of a polypeptide for screening (preferably FGK) as the index, and
(3) a screening method which uses activity change of a polypeptide for screening (preferably FGK) as the index, using the COLIA2 promoter are described below in order.

In the (1) a screening method which uses activity change of a polypeptide for screening (preferably FGK) as the index,
1) a method for screening an inverse agonist using a change of intracellular Ca²⁺ concentration as the index (to be referred to as Ca²⁺ type screening method hereinafter);
2) a method for screening an inverse agonist using a change of intracellular cAMP quantity as the index (to be referred to as cAMP type screening method hereinafter); and
3) a method for screening an inverse agonist using the GTPγS binding assay (to be referred to as GTPγS binding type screening method hereinafter) are included and these are described in order in the following.

### 1) Ca²⁺ type screening method

The Ca²⁺ type screening method of the present invention is not particularly limited, so far as it includes a step for detecting whether or not it is an inverse agonist by the Ca²⁺ type detection method of the present invention and a step for selecting an inverse agonist.

The inverse agonist can be screened by the aforementioned Ca²⁺ type detection method using reduction of Ca²⁺ concentration in the cell for Ca²⁺ type detection as the index.

For example, by allowing a test compound to undergo the reaction for a predetermined period of time and using the reduction of intracellular Ca²⁺ concentration as the index, a substance having an IC₅₀ value of 10 µM or less, preferably a substance having an IC₅₀ value of 1 µM or less, more preferably a substance having an IC₅₀ value of 0.1 µM or less can be selected as an inverse agonist. By carrying out screening of inverse agonists by the Ca²⁺ type screening method of the present invention, a substance which is useful as an agent for treating renal failure can be screened.

### 2) cAMP type screening method

The cAMP type screening method of the present invention is not particularly limited, so far as it includes a step for detecting whether or not it is an inverse agonist by the cAMP type detection method of the present invention and a step for selecting an inverse agonist.

The inverse agonist can be screened by the aforementioned cAMP type detection method using increase of intracellular cAMP concentration of the cell for cAMP type detection as the index.

Using increase of intracellular cAMP concentration as the index, a test compound having an ED₅₀ value of 10 µM or less (more preferably 1 µM or less) can be selected as a substance having inverse agonist activity.

By carrying out screening of inverse agonists by the cAMP ⁺ type screening method of the invention, a substance which is useful as an agent for treating renal failure can be screened.

### 3) GTPγS binding type screening method

The GTPγS binding type screening method of the present invention is not particularly limited, so far as it includes a step for detecting whether or not it is an inverse agonist by the GTPγS binding type detection method of the present invention and a step for selecting an inverse agonist.

The inverse agonist can be screened by the aforementioned GTPγS binding type detection method, using reduction of specific GTPγS binding by a test compound as the index.

By carrying out screening of inverse agonists by the GTPγS binding type screening method of the present invention, a substance useful as an agent for treating renal failure treating can be screened.

### (2) Method for screening a substance which inhibits CTGF expression, using inhibition of activity of a polypeptide for screening (preferably FGK) as the index

It is shown that a substance which inhibits expression of CTGF becomes an agent for treating renal failure. Accordingly, a substance which is useful as an agent for treating renal failure can be screened by a method of the present invention for screening a substance which inhibits expression of CTGF, namely, a method which has the feature comprising
a step of allowing a cell for screening expressing the CTGF promoter having a reporter gene in its downstream to contact with a test compound, and
a step of measuring the reporter activity in the aforementioned cell. More illustratively, the aforementioned screening can be carried out by the method described in Example 3 or Example 7. For example, by adding a test compound under the assay condition described in Example 3 or Example 7, a substance having an IC₅₀ value of 10 µM or less, preferably a substance having an IC₅₀ value of 1 µM or less, more preferably a substance having an IC₅₀ value of 0.1 µM or less under the condition of Example 3 or Example 7 can be selected as a substance which is useful as an agent for treating renal failure.

### (3) Screening method which uses change in activity of a polypeptide for screening (preferably FGK) as the index, using the COLIA2 promoter

A polypeptide for screening (preferably FGK) which activates the promoter of CTGF as a target of an agent for treating renal failure is useful by itself as a screening tool for an agent for treating a renal failure. The inventors of the present invention have found that FGK activates the promoter of COLIA2 as shown in Example 3 which is described later, and a screening method which uses change in FGK activity as the index using the COLIA2 promoter was established. As one of the systems in which activity inhibition of activity of FGK is detected *in vitro,* the following reporter assay system using the COLIA2 promoter can be used.

The screening method of the present invention which uses change in activity of a polypeptide for screening (preferably FGK) as the index using the COLIA2 promoter has the feature comprising
a step of allowing a cell for a screening tool expressing the COLIA2 promoter having a reporter gene in its downstream to contact with a test compound and
a step of measuring the reporter activity in the aforementioned cell. The screening method can be carried out by using the COLIA2 promoter instead of the CTGF promoter in the aforementioned method for screening a substance which inhibits CTGF expression. By carrying out the aforementioned steps and thereby selecting a substance which inhibits the reporter activity, a substance which is useful for treating renal failure can be screened. More illustratively, the aforementioned screening can be carried out by the method described in Example 3. For example, by adding a test compound under the assay condition described in Example 3, a substance having an IC₅₀ value of 10 µM or less, preferably a substance having an IC₅₀ value of 1 µM or less, more preferably a substance having an IC₅₀ value of 0.1 µM or less under the condition of Example 3 can be selected as a substance which is useful as an agent for treating renal failure.

### <Pharmaceutical composition for treating renal failure and production method thereof>

In the present invention, a pharmaceutical composition which comprises an inverse agonist for the polypeptide for a screening tool [e.g., a DNA, a protein (includes an antibody or antibody fragment), a peptide or other compound] which can be selected, for example, by the screening method of the present invention as an active ingredient is included.

Also, a method for producing a pharmaceutical composition for treating renal failure, which comprises (1) (i) a step of allowing a cell for a screening tool or its cell membrane co-expressing a chimeric G protein in which its C-terminal amino acid sequence is the amino acid sequence represented by SEQ ID NO:16, and which is a chimera of a partial polypeptide having promoting activity of phospholipase C activity of a phospholipase C activity-promoting G protein with a partial polypeptide having a Gi receptor coupling activity, to contact with a test compound, and (ii) a step of analyzing a change in activity of a polypeptide for a screening tool in the aforementioned cell, or (2) (i) a step of allowing a cell for a screening tool or its cell membrane expressing the DNA of SEQ ID NO:13 or 14 having a reporter gene in its downstream to contact with a test compound, and (ii) a step of analysis comprising measurement of the reporter activity in the aforementioned cell and then making the analyzed substance into a pharmaceutical composition is included in the present invention.

In the present invention, a method for producing a pharmaceutical composition for treating renal failure ahs the feature comprising a step of carrying out screening using the screening method of the present invention and a step of preparing a pharmaceutical composition using a substance obtained by the aforementioned screening is included.

As the active ingredient in the pharmaceutical composition of the present invention, an inverse agonist of the polypeptide for a screening tool can be used, and the aforementioned inverse agonist can be selected, for example, by the screening method of the present invention. As the inverse agonist for the polypeptide for a screening tool, for example, the compounds selected by the screening method of the present invention (cf. Examples 5 to 7 and Table 1 which are described later) can be cited. The pharmaceutical composition of the present invention is not limited to a pharmaceutical composition which comprises a substance obtained by the screening method of the present invention as the active ingredient. All of pharmaceutical compositions for treating renal failure which comprise an inverse agonist for the polypeptide for a screening tool as the active ingredient are included therein.

In this connection, confirmation of the presence of the effect of treating renal failure can be carried out by a method commonly known to those skilled in the art or a modified method thereof. The effect of treating renal failure can be detected using inhibition of tubulointerstitial fibrosis, reduction of the amount of protein in urine, amount of blood creatinine or the like as the index. For example, with regard to the confirmation of the inhibitory effect on tubulointerstitial fibrosis, it can be confirmed using the UUO model mouse described in the Non-patent Reference 15 or a non-patent reference: *Kidney International,* 2002, vol. 61, pp. 1684 - 1695, or a modified method thereof. More illustratively, it can be confirmed by the following method. Male ICR mice are anesthetized by intraperitoneally injecting pentobarbital (50 mg/kg) (i.p.) at a liquid dose of 0.1 ml/10 g. The UUO mice can be prepared by incising the left abdominal side, ligating the left urinary duct at two positions with 4-0 silk thread, cutting the space, and then stitching the incised part. The UUO mice are divided into a control group and a test compound administration group, and an agent is administered by a method corresponding to its type. After the administration (e.g., 5 days after the preparation of UUO by once a day administration), a blood sample is collected under pentobarbital anesthesia, and then left kidney (morbid state kidney) and right kidney (control kidney) are excised and their wet weights are measured. By dividing the kidney, a part thereof can be pathologically evaluated (e.g., Masson trichrome staining (collagen protein accumulated in the interstitium is stained blue)). Also, by extracting RNA from a part thereof, expression of a fibrosis marker gene (e.g., CTGF, collagen, fibronectin or the like) can be detected by a conventionally known method (e.g., northern blotting or RT-PCR). When staining of the test compound administration group is reduced (when collagen protein is reduced) in comparison with the control group by the aforementioned staining, it can be judged that the test compound has the effect of treating renal failure. Also, when expression quantity of a fibrosis marker in the test compound administration group is reduced in comparison with the control group by the aforementioned staining, it can be judged that the test compound has the effect of treating renal failure.

In addition, reduction of protein in urine can be confirmed, for example, using the 5/6 kidney excision rat described in a non-patent reference *Pharmacological Research,* 2003, vol. 47, pp. 243 - 252 or a modified method thereof.

Among the confirming methods of the aforementioned effect of treating renal failure, most desirable one is the method which uses 5/6 nephrectomized rat. More illustratively, it can be confirmed by the following method. Wister rats of 9 weeks age are anesthetized by intraperitoneally injecting pentobarbital (50 mg/kg) (i.p.). A 5/6 nephrectomized renal failure model rat can be prepared by incising the left abdominal side, excising 2/3 of the left kidney, and then excising whole portion of the right kidney one week thereafter. The 5/6 nephrectomized renal failure model rat are divided into a control group and a test compound administration group, and an agent is administered by a method corresponding to its type (e.g., once a day administration starting 2 weeks after the preparation of the 5/6 nephrectomized renal failure model rat ). In every 1 week after the operation, measurement of blood pressure and urine collection test in individual metabolism cage are carried out periodically (e.g., once a week for 10 weeks). At the time of sacrifice, blood sample is collected from the abdominal vena cava and kidney excision is carried out. The urine samples are used for the measurement of the total volume, protein concentration and the like, and the blood samples are used for that of the concentration of cholesterol, urea nitrogen, creatinine and the like, and the kidney tissue samples are used for the evaluation of pathological tissue changes and the like. For example, when the amount of protein in urine is reduced, or when urea nitrogen quantity or creatinine quantity in blood is reduced in comparison with the control group, it can be judged that the test compound has the effect of treating renal failure.

The pharmaceutical preparation which comprises a substance or an inverse agonist of the polypeptide for a screening tool [e.g., a DNA, a protein (includes an antibody or antibody fragment), a peptide or other compound], obtained by the screening method of the present invention as the active ingredient can be prepared as a pharmaceutical composition using pharmaceutically acceptable carrier, excipient and/or other additives generally used for preparation, according to the type of the aforementioned active ingredient.

An administration includes such as oral administration through tablets, pills, capsules, granules, fine subtilaes, powders and oral solutions, and parenteral administration through injections such as intravenous injections, intramuscular injections, intraarticular injections or the like, suppositories, percutaneous administration preparations and transmucosal administration preparations. Particularly in the case of peptides which are digested in the stomach, parenteral administration such as intravenous injection is desirable.

In a solid composition for oral administration, one or more active substances can be mixed with at least one inert diluent such as lactose, mannitol, glucose, microcrystalline cellulose, hydroxypropylcellulose, starch, polyvinyl pyrrolidone and aluminum magnesium silicate. As usual, the aforementioned composition may contain other additives than the inert diluent, such as a lubricant, a disintegrating agent, a stabilizing agent and a solubilizing or solubilization assisting agent. As occasion demands, tablets or pills may be coated with a sugar coating or a film of a gastric or enteric substance.

The liquid composition for oral administration can include emulsions, solutions, suspensions, syrups, or elixirs and can contain a generally used inert diluent such as purified water or ethanol. The aforementioned composition can contain additives other than the inert diluent such as a moistening agent, a suspending agent, a sweetener, an aromatic and antiseptic.

The injections for parenteral administration can include aseptic aqueous or non-aqueous solutions, suspensions or emulsions. As a diluent, the aqueous solutions or suspensions can include such as distilled water for injection and physiological saline. Examples of a diluent of the non-aqueous solutions and suspensions can include propylene glycol, polyethylene glycol, plant oil (e.g., olive oil), alcohols (e.g., ethanol), polysorbate 80 and the like. The aforementioned composition can further contain a moistening agent, an emulsifying agent, a dispersing agent, a stabilizing agent , a solubilizing or solubilization assisting agent, an antiseptic or the like. The aforementioned composition can be sterilized by filtration through a bacteria retaining filter, blending of a germicide or irradiation. Alternatively, a sterile solid composition is produced, which can be used by dissolving in sterile water or other sterile solvent for injection prior to its use.

The dose can be optionally decided by taking into consideration of strength of the activity of the active ingredient, namely a substance obtainable by the screening method of the invention, symptoms and age, sex and the like of each subject to be administered.

For example, in the case of oral administration, its dose is usually from about 0.1 to 100 mg, preferably from 0.1 to 50 mg, per day per an adult (body weight of 60 kg). In the case of parenteral administration, it is from 0.01 to 50 mg, preferably from 0.01 to 10 mg, per day, in the form of injections.

### Examples

The present invention is described in detail in the following based on examples, but the present invention is not limited by the examples. In this connection, unless otherwise noted, it is possible to carry out in accordance with conventionally known methods (Maniatis, T. *et al.*, "Molecular Cloning - A Laboratory Manual", Cold Spring Harbor Laboratory, NY, 1982, and the like).

Also, when commercially available reagents and kits are used, it is possible to carry out in accordance with manuals attached to commercial products.

### (Example 1) Isolation of gene coding for FGK

A complete cDNA coding for the FGK of the present invention was obtained by PCR using a human kidney cDNA (Clontech) as a template. The oligonucleotide represented by SEQ ID NO:3 was used as the forward primer, and the oligonucleotide represented by SEQ ID NO:4 as the reverse primer (a *Xba*I site was added to each 5' end). A DNA polymerase (Pyrobest DNA Polymerase; Takara Shuzo) was used in the PCR, and a cycle of 98°C (30 seconds)/55°C (30 seconds)/72°C (2 minutes) was repeated for 30 cycles in the presence of 5% DMSO. As a result, a DNA fragment of about 1.0 kbp was amplified. This fragment was digested with *Xba*I and then cloned using pEF-BOS-dhfr plasmid (Mizushima, S. and Nagata, S. (1990) *Nucleic Acids Res.,* **18,** 5322). The thus constructed plasmid was named pEF-BOS-dhfr-FGK. When nucleotide sequence of the obtained clone was analyzed by dideoxy terminator method using AB1377 DNA Sequencer (Applied Biosystems), the nucleotide sequence represented by SEQ ID NO:1 was obtained. The sequence has an open reading frame (ORF) of 993 bases. An amino acid sequence (330 amino acids) deduced from the ORF is shown in SEQ ID NO:2.

### (Example 2) Preparation of a reporter plasmid using human connective tissue growth factor (CTGF) promoter and a reporter plasmid using human type I collagen alpha 2 subunit (COLIA2) promoter

A human genomic DNA derived from total blood (Human Genomic DNA; Clontech) was used as the template DNA for the amplification of promoter regions of human CTGF and human COLIA2 genes. For the amplification of the CTGF promoter region, an oligonucleotide consisting of the nucleotide sequence represented by SEQ ID NO:5 was used as the forward primer, and an oligonucleotide consisting of the nucleotide sequence represented by SEQ ID NO:6 was used as the reverse primer. Also, for the amplification of the COLIA2 promoter region, an oligonucleotide consisting of the nucleotide sequence represented by SEQ ID NO:7 was used as the forward primer, and an oligonucleotide consisting of the nucleotide sequence represented by SEQ ID NO:8 was used as the reverse primer. In the PCR, a DNA polymerase (Pfu turbo DNA polymerase; Stratagene) was used, and after heating at 94°C (2 minutes), a cycle of 94°C (30 seconds)/58°C (30 seconds)/72°C (2 minutes) was repeated 35 times. As a result, a DNA fragment of about 1.2 kbp in the case of the CTGF promoter, or of about 0.4 kb in the case of the COLIA2 promoter, was amplified. Thereafter, the respective amplified products were separated by 1% agarose gel and purified using a spin column (QIAquick Gel Extraction Kit; Qiagen), and then PCR was again carried out using each of them as the template. For the amplification of the CTGF promoter region, an oligonucleotide consisting of the nucleotide sequence represented by SEQ ID NO:9 was used as the forward primer, and an oligonucleotide consisting of the nucleotide sequence represented by SEQ ID NO:10 was used as the reverse primer. Also, for the amplification of the COLIA2 promoter region, an oligonucleotide consisting of the nucleotide sequence represented by SEQ ID NO:11 was used as the forward primer, and an oligonucleotide consisting of the nucleotide sequence represented by SEQ ID NO:12 was used as the reverse primer. In this case, a *Kpn*I recognizing sequence was added to the 5' end of the each forward primer, and a *Bgl*II recognizing sequence was added to the 5' end of each reverse primer. In the PCR, a DNA polymerase (Pfu turbo DNA polymerase; Stratagene) was used, and after heating at 94°C (2 minutes), a cycle of 94°C (30 seconds)/58°C (30 seconds)/72°C (2 minutes) was repeated for 30 times. Thereafter, the respective amplified products were separated by 1% agarose gel; purified using a spin column (QIAquick Gel Extraction Kit; Qiagen); digested with *Kpn*I and *Bgl*II; inserted into a plasmid for reporter expression (Pickagene Basic Vector 2; Toyo Ink); and then cloned. Nucleotide sequences of the obtained clones were analyzed by dideoxy terminator method using AB13770 DNA Sequencer (Applied Biosystems), and a clone coincided with a known CTGF promoter sequence (GenBank accession No. AL354866) or COLIA2 promoter sequence (GenBank accession No. AF004877) was respectively selected. The reporter plasmids obtained by the above respectively contained from -1,129 bp to +24 bp (SEQ ID NO:13) of the human CTGF promoter or from -337 bp to +22 bp (SEQ ID NO:14) of the human COLIA2 promoter, and respectively named pCTGF-luc and pCOLIA2-luc.

### (Example 3) Construction of reporter assay system of CTGF promoter and COLIA2 promoter and effects by FGK transfection

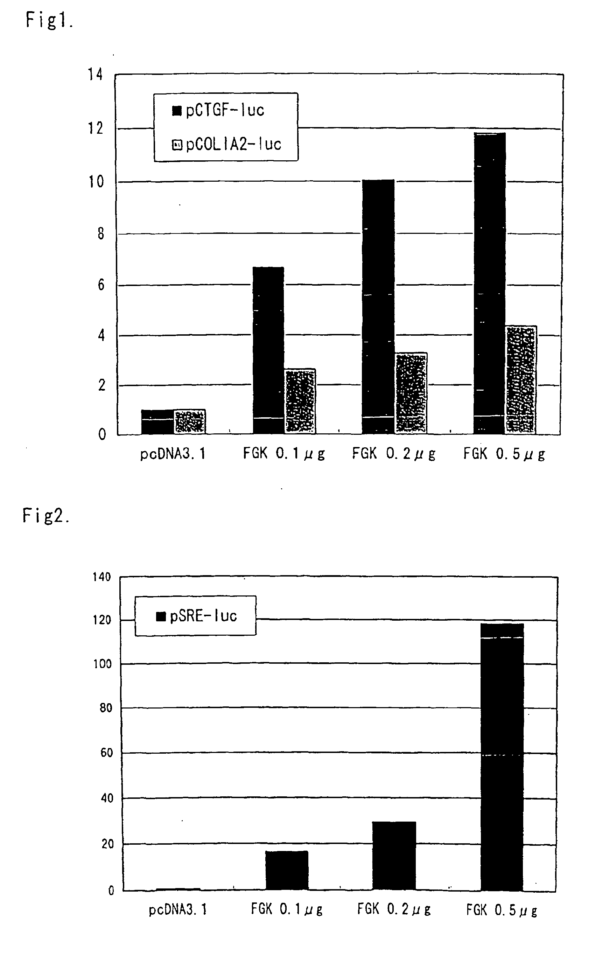

Using Dulbecco's modified Eagle's medium (DMEM) containing 10% fetal bovine serum, HEK293 cells (obtained from ATCC) were dispensed in 1 × 10⁵ cells per well portions into a 24-well plate (Asahi Technoglass) and cultured at 37°C for 24 hours. Transfection of 0.1 µg of a reporter plasmid and 0.5 µg at the maximum of a human FGK expression plasmid which is 0.6 µg in total per well was carried out using a transfection reagent (FuGENE6; Boehringer-Roche) in accordance with the instructions attached thereto. In this case, the amount of the transfected genes in each of the all wells was adjusted to 0.6 µg in total per well by adding a plasmid vector (pcDNA3.1; Invitrogen). Cells transfected with the pcDNA3.1 vector alone without containing FGK, together with the reporter plasmid, was used as the control. The medium was discarded 24 hours after transfection, and the residue was lysed by adding 100 µl per well of a cell lysis buffer. A 50 µl portion of the lysate was mixed with 100 µl of a luciferin substrate solution and allowed to undergo the reaction to be measured using a luminometer (ML3000; Dynatech Laboratories). The relative activity to the luciferase activity of the control (control is defined as 1) is shown in Fig. 1. Activation of the CTGF promoter and COLIA2 promoter by the expression of FGK was observed. Additionslly, dose-dependent activation by FGK was also observed. Based on the above, it was revealed that FGK induces expression of CTGF and type I collagen.

By using the assay system, a substance which inhibits CTGF expression and a substance which is useful as an agent for treating renal failure can be screened.

### (Example 4) Construction of screening system which uses FGK activity change as the index

An expression plasmid used in the Example for expressing a chimeric protein of Gq and Gi was prepared by cloning, a gene (hereinafter, to be referred to as Gqi gene) constructed by replacing 5 amino acids in the Gq C-terminal side (Glu-Tyr-Asn-Leu-Val; the amino acid sequence represented by SEQ ID NO:15) with 5 amino acids in the Gi C-terminal side (Asp-Cys-Gly-Leu-Phe; the amino acid sequence represented by SEQ ID NO:16) in accordance with the method of Conklin, B.R. *et al*. (*Nature,* **363,** 274 - 276, 1993) into the plasmid pEF-BOS-dhfr. The constructed plasmid was named pEF-BOS-Gqi.

As the illustrative method, firstly, using Dulbecco's modified Eagle's medium (DMEM) containing 10% fetal bovine serum,, HEK293 cells (obtained from ATCC) were dispensed in 1 × 10⁵ cells per well portions into a 24-well plate (Asahi Technoglass) and cultured for 24 hours. Then transfection of 0.05 µg of a reporter plasmid pSRE-luc in which a serum response sequence was inserted into upstream of a luciferase gene (PathDetect™ SRE cis-Reporting System; Stratagene), 0.05 µg of pEF-BOS-Gqi and 0.5 µg at the maximum of a human FGK expression plasmid which is 0.6 µg in total per well was carried out using a transfection reagent (FuGENE6; Boehringer-Roche). Cells transfected with the pcDNA3.1 vector alone without containing FGK, together with the reporter plasmid and pEF-BOS-Gqi, was used as the control. Other conditions and measurement of the reporter activity were as described in Example 3. The relative activity to the luciferase activity of the control (control is defined as 1) is shown in Fig. 2. Increase in the reporter activity by the expression of FGK was observed. Additionally, dose-dependent activation by FGK was also observed. Based on these results above, screening of a substance which inhibits FGK activity, namely a substance which is useful as an agent for treating renal failure by the assay system becomes possible.

### (Example 5) Screening of a substance which inhibits FGK activity

Screening of a test substance was carried out using the screening system which uses changes in FGK activity as the index, constructed in Example 4.

Using 10% fetal bovine serum-containing Dulbecco's modified Eag;e' smedium (DMEM), HEK293 cells(obtained from ATCC) were inoculated in 1 × 10⁴ cells per well portions into a 96-well plate (Asahi Technoglass) and cultured at 37°C for 24 hours. Transfection of 0.01 µg of the reporter plasmid pSRE-luc (Example 4), 0.03 µg of pEF-BOS-Gqi and 0.02 µg of a human FGK expression plasmid which is 0.06 µg in total per well was carried out using a transfection reagent (FuGENE6; Boehringer-Roche). After 6 hours of incubation at 37°C, a dimethyl sulfoxide solution of each test compound was added to become a final concentration of 30 µM, 10 µM, 3 µM, 1 µM, 0.3 µM or 0.1 µM, and then incubated at 37°C for additional 24hours. Thereafter, the reporter activity was measured to determine 50% activity inhibition concentration (IC₅₀) of each test compound. As a control of the compound treatment, reporter activity when the same volume of dimethyl sulfoxide alone was added was regarded as 100%. Other conditions and measurement of reporter activity were as described in Example 4. Seven different compounds can be obtained when a substance which inhibits the FGK activity in cells expressing FGK (a substance having an IC₅₀ value of 10 µM or less) were selected as inverse agonists for FGK. Structures, activities and suppliers of these compounds are shown in Table 1.

### (Example 6) Detection of the inhibition of CTGF expression by a substance which inhibits FGK activity

Primary culture cells of human proximal renal tubule epithelium (RPTEC; Asahi Technoglass) were plated at 2 × 10⁵ cells per well into 12-well plates (Asahi Technoglass) using a proliferation medium for kidney epithelial cell use (Burette Kit REGM; Asahi Technoglass). After incubation for 24hours at 37°C, each of the test compounds obtained in Example 5 (substances which inhibit FGK activity, namely FGK inverse agonists) was added thereto to a final concentration of 10 µM, and then incubated for additional 24 hours at 37°C.. Thereafter, the cells were collected and the total RNA was purified using a spin column (RNeasy Mini Kit; Qiagen). Reverse transcription reaction was carried out using 1.5 µg of the total RNA and a reverse transcriptase (PowerScript Transcriptase; BD Biosciences) to obtain cDNA samples. Using each of them as the template, expressed amounts of CTGF and a housekeeping gene of glyceraldehyde 3-phosphate dehydrogenase (to be referred to as G3PDH hereinafter) were measured by real time PCR using PRISM 7700 Sequence Detector (PERKIN ELMER). In the PCR, SYBR Green PCR Master Mix (Applied Biosystems) was used, and after heating at 50°C (2 minutes) and 95°C (10 minutes) , a cycle of 95°C (15 seconds)/60°C (60 seconds) was repeated for 40 times. For the detection of CTGF, an oligonucleotide represented by SEQ ID NO:17 was used as the forward primer, and an oligonucleotide represented by SEQ ID NO:18 was used as the reverse primer. For the detection of G3PDH, an oligonucleotide represented by SEQ ID NO:19 was used as the forward primer and an oligonucleotide represented by SEQ ID NO:20 was used as the reverse primer. A value calculated by dividing the expressed amount of CTGF by the expressed amount of G3PDH, both obtained by the PCR, was defined as the CTGF expression quantity per unit RNA quantity. Also, as a control of the compound treatment, the CTGF expression quantity per unit RNA quantity when the same volume of dimethyl sulfoxide alone was added was regarded as 100%. The inhibitory activity of CTGF expression obtained by the assay (inhibitory activity at a compound concentration of 10 µM) is shown in Table 1. It was able to confirm that the substances obtained using the inhibition of FGK activity as the index surely inhibit CTGF expression.

### (Example 7) Screening of a compound which inhibits CTGF promoter activation by FGK

Screening of a test compound constructed in Example 3 was carried out using the CTGF promoter activation system by FGK.

HEK293 cells (obtained from ATCC) were plated at 1 × 10⁴ cells per well into 96-well plates (Asahi Technoglass) in Dulbecco's modified Eagle's medium containing 10% fetal bovine serum (DMEM). After incubation for 24hours at 37°C, cells were transfected with 0.02 µg of pCTGF-luc (cf. Example 2) and 0.02 µg of a human FGK expression plasmid which is 0.04 µg in total per well was carried out using a transfection reagent (FuGENE6; Boehringer-Roche). After incubation for 6hours at 37°C, a dimethyl sulfoxide solution of each test compound was added thereto to become a final concentration of 30 µM, 10 µM, 3 µM, 1 µM, 0.3 µM or 0.1 µM, and then incubated at 37°C for additional 24 hours. Thereafter, the reporter activity was measured to determine 50% activity inhibition concentration (IC₅₀) of each test compound. As a control of the compound treatment, reporter activity when the same volume of dimethyl sulfoxide alone was added was regarded as 100%. Other conditions and measurement of reporter activity were as described in Example 3. Substances which inhibit CTGF promoter activation by FGK were selected, and the substances having an IC₅₀ value of 10 µM or less were shown in Table 1. It was confirmed that a substance which inhibits CTGF expression by the assay system, namely a substance useful as a renal failure treating agent can be certainly be screened.

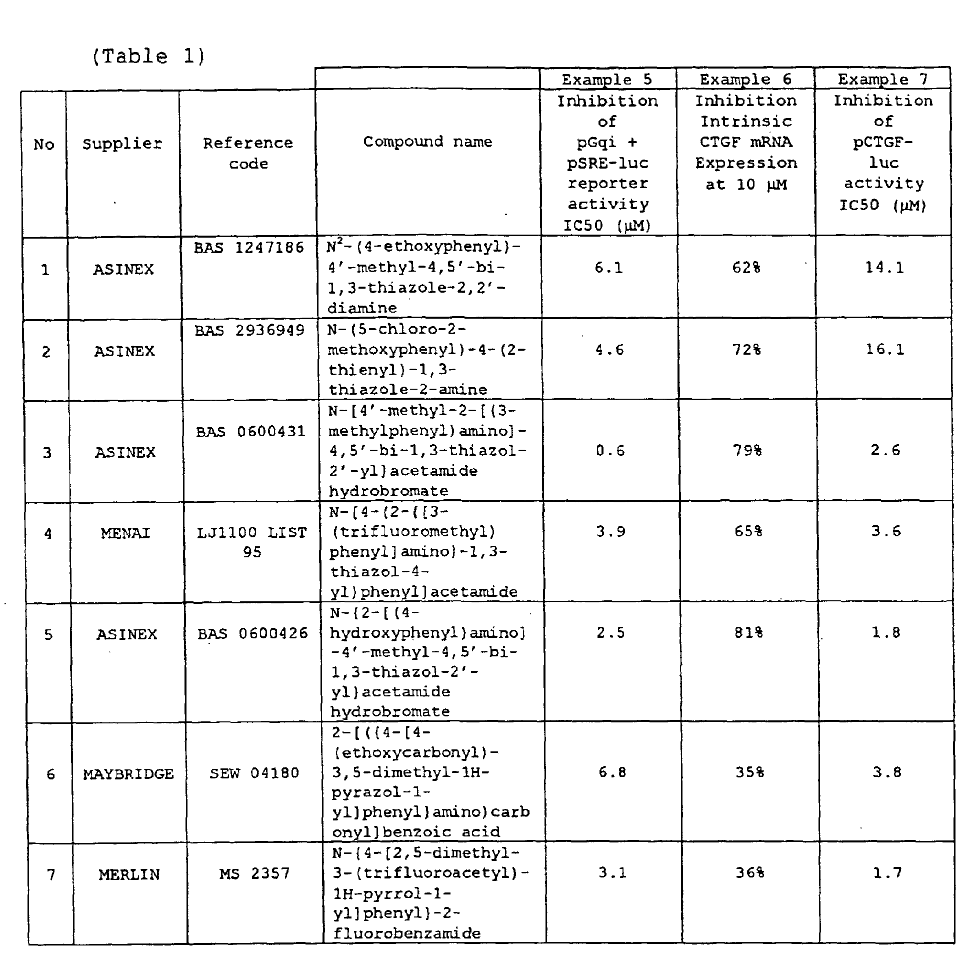

### Industrial Applicability

It was found that FGK as one of the a screening tools of the invention activates the promoter of CTGF. It is known that CTGF is a therapeutic target for renal failure, by the use of the polypeptide as a screening tool of the present invention and/or a cell expressing the aforementioned polypeptide, construction of a method for screening a substance which inhibits CTGF expression using inhibition of the aforementioned polypeptide as the index, namely a method for screening an agent for treating renal failure based on the inhibition of CTGF expression by selecting an inhibitor of the aforementioned polypeptide and a method for screening an agent for treating renal failure by selecting an inverse agonist for the aforementioned polypeptide is possible.

In addition, a pharmaceutical composition for treating renal failure can be produced by using a substance as the active ingredient which is obtainable by the screening method of the present invention and making it into a pharmaceutical composition using a carrier, an excipient and/or other additives.

### Sequence Listing Free Text

An explanation of the "Artificial Sequence" is described in the numerical entry <223> of the Sequence Listing. Illustratively, respective nucleotide sequences represented by the sequences of SEQ ID NOs:3, 4 and 9 to 12 are artificially synthesized primer sequences.

Although the invention has been described in the foregoing based on the specific embodiments, various changes and modifications obvious to those skilled in the art are included within the scope of the present invention.

## Claims

1. A screening tool for an agent for treating renal failure, which is a polypeptide consisting of the amino acid sequence represented by SEQ ID NO:2, or a polypeptide comprising an amino acid sequence represented by SEQ ID NO:2 in which from 1 to 10 amino acids are deleted, substituted and/or inserted and which is capable of activating CTGF promoter.

2. The screening tool for an agent for treating renal failure, which is a cell expressing the polypeptide described in claim 1.

3. A method for detecting whether or not a test compound is an inverse agonist, which comprises
a step of allowing the cell described in claim 2 co-expressing a chimeric G protein in which C-terminal amino acid sequence is the amino acid sequence represented by SEQ ID NO:16, and which is a chimera of a partial polypeptide having promoting activity of phospholipase C activity of a phospholipase C activity-promoting G protein with a partial polypeptide having a Gi receptor coupling activity, to contact with a test compound, and
a step of analyzing a change in activity of the polypeptide described in claim 1 in said cell.

4. A method for screening an agent for treating renal failure, which comprises
a step of allowing the cell described in claim 2 co-expressing a chimeric G-protein in which C-terminal amino acid sequence is the amino acid sequence represented by SEQ ID NO:16, and which is a chimera of a partial polypeptide having promoting activity of phospholipase C activity of a phospholipase C activity-promoting G protein with a partial polypeptide having a Gi receptor coupling activity, to contact with a test compound, and
a step of analyzing a change in activity of the polypeptide described in claim 1 in said cell.

5. A method for screening a substance inhibiting expression of CTGF, which comprises
a step of allowing the cell described in claim 2 expressing the DNA of SEQ ID NO:13 having a reporter gene in downstream to contact with a test compound, and
a step of measuring the reporter activity in said cell.

6. The screening method according to claim 5, wherein the substance inhibiting expression of CTGF is an agent for treating renal failure.

7. A method for screening an agent for treating renal failure, which comprises
a step of allowing the cell described in claim 2 expressing the DNA of SEQ ID NO:14 having a reporter gene in downstream to contact with a test compound, and
a step of measuring the reporter activity in said cell.

8. A pharmaceutical composition for treating renal failure, which comprises an inverse agonist for the polypeptide described in claim 1.

9. A pharmaceutical composition for treating renal failure, which comprises a substance obtainable by the method according to one of claim 4 to claim 7.

10. A method for producing a pharmaceutical composition for treating renal failure, which comprises
a step of screening using the method according to one of claim 4 to claim 7, and
a step of preparing a pharmaceutical composition using a substance obtained by said screening.

11. A method for treating renal failure, which comprises administering an effective amount of an inverse agonist for the polypeptide described in claim 1 and/or a substance obtaiable by the method according to one of claim 4 to claim 7 to a subject in need of the treatment of renal failure.

12. Use of an inverse agonist for the polypeptide described in claim 1 and/or a substance obtainable by the method according to one of claim 4 to claim 7 for the manufacture of a pharmaceutical composition for treating renal failure.


SEQUENCE LISTING

<110>  Yamanouchi Pharmaceutical Co.,Ltd.

<120>  Methods for screening an agent to treat renal failure

<130>  Y0358-PCT

<150>  JP2002-298958
<151>  2002-10-11

<160>  20

<170>  PatentIn version 3.1

<210>  1
<211>  993
<212>  DNA
<213>  Homo sapiens

<220>
<221>  CDS
<222>  (1)..(990)
<223>  inventor:Enjo, Kentaro; Kuromitsu, Sadao

<400>  1
atg gca tgg aat gca act tgc aaa aac tgg ctg gca gca gag gct gcc      48
Met Ala Trp Asn Ala Thr Cys Lys Asn Trp Leu Ala Ala Glu Ala Ala
1               5                   10                  15

ctg gaa aag tac tac ctt tcc att ttt tat ggg att gag ttc gtt gtg      96
Leu Glu Lys Tyr Tyr Leu Ser Ile Phe Tyr Gly Ile Glu Phe Val Val
            20                  25                  30

gga gtc ctt gga aat acc att gtt gtt tac ggc tac atc ttc tct ctg      144

Gly Val Leu Gly Asn Thr Ile Val Val Tyr Gly Tyr Ile Phe Ser Leu
        35                  40                  45

aag aac tgg aac agc agt aat att tat ctc ttt aac ctc tct gtc tct 192
Lys Asn Trp Asn Ser Ser Asn Ile Tyr Leu Phe Asn Leu Ser Val Ser
    50                  55                  60

gac tta gct ttt ctg tgc acc ctc ccc atg ctg ata agg agt tat gcc 240
Asp Leu Ala Phe Leu Cys Thr Leu Pro Met Leu Ile Arg Ser Tyr Ala
65                  70                  75                  80

aat gga aac tgg ata tat gga gac gtg ctc tgc ata agc aac cga tat 288
Asn Gly Asn Trp Ile Tyr Gly Asp Val Leu Cys Ile Ser Asn Arg Tyr
                85                  90                  95

gtg ctt cat gcc aac ctc tat acc agc att ctc ttt ctc act ttt atc 336
Val Leu His Ala Asn Leu Tyr Thr Ser Ile Leu Phe Leu Thr Phe Ile
            100                 105                 110

agc ata gat cga tac ttg ata att aag tat cct ttc cga gaa cac ctt 384
Ser Ile Asp Arg Tyr Leu Ile Ile Lys Tyr Pro Phe Arg Glu His Leu
        115                 120                 125

ctg caa aag aaa gag ttt gct att tta atc tcc ttg gcc att tgg gtt 432
Leu Gln Lys Lys Glu Phe Ala Ile Leu Ile Ser Leu Ala Ile Trp Val
    130                 135                 140

tta gta acc tta gag tta cta ccc ata ctt ccc ctt ata aat cct gtt 480
Leu Val Thr Leu Glu Leu Leu Pro Ile Leu Pro Leu Ile Asn Pro Val
145                 150                 155                 160

ata act gac aat ggc acc acc tgt aat gat ttt gca agt tct gga gac 528
Ile Thr Asp Asn Gly Thr Thr Cys Asn Asp Phe Ala Ser Ser Gly Asp
                165                 170                 175

ccc aac tac aac ctc att tac agc atg tgt cta aca ctg ttg ggg ttc 576

Pro Asn Tyr Asn Leu Ile Tyr Ser Met Cys Leu Thr Leu Leu Gly Phe
            180                 185                 190

ctt att cct ctt ttt gtg atg tgt ttc ttt tat tac aag att gct ctc 624
Leu Ile Pro Leu Phe Val Met Cys Phe Phe Tyr Tyr Lys Ile Ala Leu
        195                 200                 205

ttc cta aag cag agg aat agg cag gtt gct act gct ctg ccc ctt gaa 672
Phe Leu Lys Gln Arg Asn Arg Gln Val Ala Thr Ala Leu Pro Leu Glu
    210                 215                 220

aag cct ctc aac ttg gtc atc atg gca gtg gta atc ttc tct gtg ctt 720
Lys Pro Leu Asn Leu Val Ile Met Ala Val Val Ile Phe Ser Val Leu
225                 230                 235                 240

ttt aca ccc tat cac gtc atg cgg aat gtg agg atc gct tca cgc ctg 768
Phe Thr Pro Tyr His Val Met Arg Asn Val Arg Ile Ala Ser Arg Leu
                245                 250                 255

ggg agt tgg aag cag tat cag tgc act cag gtc gtc atc aac tcc ttt 816
Gly Ser Trp Lys Gln Tyr Gln Cys Thr Gln Val Val Ile Asn Ser Phe
            260                 265                 270

tac att gtg aca cgg gct ttg ggc ttt ctg aac agt gtc atc aac cct 864
Tyr Ile Val Thr Arg Ala Leu Gly Phe Leu Asn Ser Val Ile Asn Pro
        275                 280                 285

gtc ttc tat ttt ctt ttg gga gat cac ttc agg gac atg ctg atg aat 912
Val Phe Tyr Phe Leu Leu Gly Asp His Phe Arg Asp Met Leu Met Asn
    290                 295                 300

caa ctg aga cac aac ttc aaa tcc ctt aca tcc ttt agc aga tgg gct 960
Gln Leu Arg His Asn Phe Lys Ser Leu Thr Ser Phe Ser Arg Trp Ala
305                 310                 315                 320

cat gaa ctc cta ctt tca ttc aga gaa aag tga 993

His Glu Leu Leu Leu Ser Phe Arg Glu Lys
                325                 330

<210>  2
<211>  330
<212>  PRT
<213>  Homo sapiens

<400>  2

Met Ala Trp Asn Ala Thr Cys Lys Asn Trp Leu Ala Ala Glu Ala Ala
1               5                   10                  15

Leu Glu Lys Tyr Tyr Leu Ser Ile Phe Tyr Gly Ile Glu Phe Val Val
            20                  25                  30

Gly Val Leu Gly Asn Thr Ile Val Val Tyr Gly Tyr Ile Phe Ser Leu
        35                  40                  45

Lys Asn Trp Asn Ser Ser Asn Ile Tyr Leu Phe Asn Leu Ser Val Ser
    50                  55                  60

Asp Leu Ala Phe Leu Cys Thr Leu Pro Met Leu Ile Arg Ser Tyr Ala
65                  70                  75                  80

Asn Gly Asn Trp Ile Tyr Gly Asp Val Leu Cys Ile Ser Asn Arg Tyr
                85                  90                  95

Val Leu His Ala Asn Leu Tyr Thr Ser Ile Leu Phe Leu Thr Phe Ile

            100                 105                 110

Ser Ile Asp Arg Tyr Leu Ile Ile Lys Tyr Pro Phe Arg Glu His Leu
        115                 120                 125

Leu Gln Lys Lys Glu Phe Ala Ile Leu Ile Ser Leu Ala Ile Trp Val
    130                 135                 140

Leu Val Thr Leu Glu Leu Leu Pro Ile Leu Pro Leu Ile Asn Pro Val
145                 150                 155                 160

Ile Thr Asp Asn Gly Thr Thr Cys Asn Asp Phe Ala Ser Ser Gly Asp
                165                 170                 175

Pro Asn Tyr Asn Leu Ile Tyr Ser Met Cys Leu Thr Leu Leu Gly Phe
            180                 185                 190

Leu Ile Pro Leu Phe Val Met Cys Phe Phe Tyr Tyr Lys Ile Ala Leu
        195                 200                 205

Phe Leu Lys Gln Arg Asn Arg Gln Val Ala Thr Ala Leu Pro Leu Glu
    210                 215                 220

Lys Pro Leu Asn Leu Val Ile Met Ala Val Val Ile Phe Ser Val Leu
225                 230                 235                 240

Phe Thr Pro Tyr His Val Met Arg Asn Val Arg Ile Ala Ser Arg Leu

                245                 250                 255

Gly Ser Trp Lys Gln Tyr Gln Cys Thr Gln Val Val Ile Asn Ser Phe
            260                 265                 270

Tyr Ile Val Thr Arg Ala Leu Gly Phe Leu Asn Ser Val Ile Asn Pro
        275                 280                 285

Val Phe Tyr Phe Leu Leu Gly Asp His Phe Arg Asp Met Leu Met Asn
    290                 295                 300

Gln Leu Arg His Asn Phe Lys Ser Leu Thr Ser Phe Ser Arg Trp Ala
305                 310                 315                 320

His Glu Leu Leu Leu Ser Phe Arg Glu Lys
                325                 330

<210> 3
<211> 29
<212> DNA
<213> Artificial

<220>
<223> Desctiption of Artificial Sequence:an artificially systhesized
primer sequence

<400> 3
ggtctagaat ggcatggaat gcaacttgc 29

<210> 4
<211> 32
<212> DNA
<213> Artificial

<220>
<223> Desctiption of Artificial Sequence:an artificially systhesized
primer sequence

<400> 4
ggtctagatt atcacttttc tctgaatgaa ag 32

<210> 5
<211> 19
<212> DNA
<213> Homo sapiens

<400> 5
tcaggctgca tgttccttg 19

<210> 6
<211> 19
<212> DNA
<213> Homo sapiens

<400> 6
tcctctcagc ggggaagag 19

<210> 7
<211> 21
<212> DNA
<213> Homo sapiens

<400>  7
gacgtgtccc atagtgtttc c                                                21

<210>  8
<211>  18
<212>  DNA
<213>  Homo sapiens

<400>  8
tcctgctgcc gtggtgct                                                    18

<210>  9
<211>  27
<212>  DNA
<213>  Artificial

<220>
<223>  Desctiption of Artificial Sequence:an artificially systhesized
                                                    primer sequence

<400>  9
ggggtacctc aggctgcatg ttccttg                                          27

<210>  10
<211>  28
<212>  DNA
<213>  Artificial

<220>
<223>  Desctiption of Artificial Sequence:an artificially systhesized
                                                    primer sequence

<400>  10

ggaagatctt cctctcagcg gggaagag 28

<210> 11
<211> 29
<212> DNA
<213> Artificial

<220>
<223> Desctiption of Artificial Sequence:an artificially systhesized
primer sequence

<400> 11
ggggtaccga cgtgtcccat agtgtttcc 29

<210> 12
<211> 27
<212> DNA
<213> Artificial

<220>
<223> Desctiption of Artificial Sequence:an artificially systhesized
primer sequence

<400> 12
ggaagatctt cctgctgccg tggtgct 27

<210> 13
<211> 1159
<212> DNA
<213> Homo sapiens

<400> 13
tcaggctgca tgttccttgg gtaatgagaa gtcacaatca ctattcatag atgtgtgggg 60

agtcactaaa aatatattat tcactgtcaa tcttagttta tatccagata caacagggta 120
cactgctctt gtaatggaat cagacttctt attttaacaa gacaaaccaa atccaatcca 180
catttgaaga ttataggttt taatataaga aaatgcactc atttctcaaa gaccctagtg 240
aagctgtgtt taaatgctcc taggtgaacc ccctttgcat cccagtgttc ccaccctgac 300
acccagagcc cctacctacc caacacagaa tcatttgctc tgatagaaca atggatccct 360
ttttctggaa acattgatgg ccactcctcc cttgtccttg cctatataaa actcctacat 420
atattaagag aaaactaagc aagagttttg gaaatctgcc ccaggagact gcatcctgag 480
tcacacgcgt ctttgttctc tttcttgtcc caaaaccgtt acctcaagtg acaaatgatc 540
aaatctcaaa tatagaattc agggttttac aggtaggcat cttgaggatt tcaaatggtt 600
aaaagcaact cactcctttt ctactctttg gagagtttca agagcctata gcctctaaaa 660
cgcaaatcat tgctaagggt tgggggggag aaaccttttc gaatttttta ggaattcctg 720
ctgtttgcct cttcagctac ctacttccta aaaaggatgt atgtcagtgg acagaacagg 780
gcaaacttat tcgaaaaaga aataagaaat aattgccagt gtgtttataa atgatatgaa 840
tcaggagtgg tgcgaagagg atagggaaaa aaaaattcta tttggtgctg gaaatactgc 900
gctttttttt ttcctttttt tttttttctg tgagctggag tgtgccagct ttttcagacg 960
gaggaatgct gagtgtcaag gggtcaggat caatccggtg tgagttgatg aggcaggaag 1020
gtggggagga atgcgaggaa tgtccctgtt tgtgtaggac tccattcagc tcattggcga 1080
gccgcggccg cccggagcgt ataaaagcct cgggccgccc gccccaaact cacacaacaa 1140

ctcttccccg ctgagagga                                                    1159

<210>  14
<211>  355
<212>  DNA
<213>  Homo sapiens

<400>  14
gacgtgtccc atagtgtttc caaacttgga aagggcgggg gagggcggga ggatgcggag      60

ggcggaggta tgcagacaac gagtcagagt ttccccttga aagcctcaaa agtgtccacg     120

tcctcaaaaa gaatggaacc aatttaagaa gccagccccg tggccacgtc ccttccccca     180

ttcgctccct cctctgcgcc cccgcaggct cctcccagct gtggctgccc gggcccccag     240

ccccagccct cccattggtg gaggcccttt tggaggcacc ctagggccag ggaaactttt     300

gccgtataaa tagggcagat ccgggcttta ttattttagc accacggcag cagga          355

<210>  15
<211>  5
<212>  PRT
<213>  Homo sapiens

<400>  15

Glu Tyr Asn Leu Val
1               5

<210>  16
<211>  5

<212>  PRT
<213>  Homo sapiens

<400>  16

Asp Cys Gly Leu Phe
1               5


<210>  17
<211>  19
<212>  DNA
<213>  Homo sapiens

<400>  17
taccaatgac aacgcctcc                                                    19


<210>  18
<211>  20
<212>  DNA
<213>  Homo sapiens

<400>  18
atgtcttcat gctggtgcag                                                   20


<210>  19
<211>  18
<212>  DNA
<213>  Homo sapiens

<400>  19
gggaaggtga aggtcgga                                                     18

<210> 20
<211> 17
<212> DNA
<213> Homo sapiens

<400> 20
gcagccctgg tgaccag 17